# EUROPÄISCHE PATENTANMELDUNG

(11) **EP 4 375 269 A1**
(43) Veröffentlichungstag der Anmeldung: **29.05.2024**
(21) Anmeldenummer: 22209330.4
(22) Anmeldetag: 24.11.2022
(51) Int. Cl.: C07C 209/84, C07C 211/09

(54) **VERFAHREN ZUR TROCKNUNG VON 1,5-DIAMINOPENTAN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Trocknung von 1,5-Diaminopentan umfassend die folgenden Schritte:
a) Bereitstellen mindestens eines Stoffgemisches enthaltend 1,5-Diaminopentan und Wasser,
b) Destillieren des Stoffgemisches in mindestens einer Destillationskolonne unter Erhalt von

Brüden sowie einer getrockneten 1,5-Diaminopentanzusammensetzung, dadurch gekennzeichnet, dass die Destillation in Schritt b) azeotrop unter Verwendung eines Schleppmittels enthaltend Monochlorbenzol durchgeführt wird.

## Beschreibung

Die Erfindung betrifft ein Verfahren zur Trocknung von 1,5-Diaminopentan umfassend die folgenden Schritte: a) Bereitstellen mindestens eines Stoffgemisches enthaltend 1,5-Diaminopentan und Wasser und b) Destillieren des Stoffgemisches in mindestens einer Destillationskolonne unter Erhalt einer getrockneten 1,5-Diaminopentanzusammensetzung sowie ein Verfahren zur Herstellung von 1,5-Diisocyanatopentan und die Verwendung des 1,5-Diisocyanatopentans zur Herstellung von oligomeren Polyisocyanaten.

1,5-Diaminopentan (im Folgenden auch als "Pentamethylendiamin", "1,5-Pentandiainin" oder "PDA" bezeichnet) ist ein wichtiges Ausgangsmaterial zur Herstellung von Polymeren. Es wird beispielsweise zur Herstellung von Polyamiden durch Polykondensation des Diamins mit Dicarbonsäuren verwendet. So entsteht beispielsweise Polyamid-5.6 (PA56) aus 1,5-Diaminopentan und Adipinsäure. Eine weitere wichtige Anwendung ist die Phosgenierung des Diamins zu Pentamethylendiisocyanat (im Folgenden auch als "1,5-Diisocyanatopentan", 1,5-Pentandiisocyanat" oder "PDI" bezeichnet), welches dann in einem weiteren Schritt beispielsweise zu Polyurethanen, Polyharnstoffen oder Polyisocyanuraten umgesetzt werden kann. Die Phosgenierung stellt hierbei besondere Anforderungen an die Reinheit des aliphatischen Diamins. Da unter Phosgenierungsbedingungen aus Wasser und Phosgen korrosive Salzsäure entsteht, wird für Amine, die in Phosgenierungen eingesetzt werden, meist ein sehr geringer Wassergehalt spezifiziert. Für PDA zur Herstellung von Pentamethylendiisocyanat wird in der WO2016042125 ein Wassergehalt von <500 ppm genannt.

Da die Anforderungen an den Wassergehalt in der Polyamid-Herstellung weniger streng sind, enthält kommerziell verfügbares PDA häufig mehr als 500 ppm Wasser und muss entsprechend vor Verwendung in der Isocyanatherstellung weiter getrocknet werden. Aufgrund der hygroskopischen Eigenschaften von PDA und der damit verbundenen starken Affinität zu Wasser, ist eine destillative Trocknung von PDA zwar möglich, aber mit Problemen wie beispielsweise Ausbeuteverlusten durch thermische Zersetzung des PDA verbunden.

Es ist dem Fachmann allgemein bekannt, organische Substanzen mittels azeotroper Destillation zu trocknen. Gebräuchliche Schleppmittel sind dabei Stoffe, die mit Wasser ein Azeotrop bilden und in der Kälte möglichst wenig mit Wasser mischbar sind, wie beispielsweise Toluol, Xylol oder Chloroform.

Dieser Ansatz wird auch in der CN216571619 U aufgegriffen. Dort wird zur Lösung des Problems eine Azeotropdestillation in Gegenwart von o-Dichlorbenzol (im Folgenden auch als "ODB" bezeichnet) beschrieben, welches ein Azeotrop mit Wasser bildet und so dessen destillative Abtrennung vom PDA erleichtert. Auf diese Weise kann die Temperatur im Sumpf der Destillationsvorrichtung geringgehalten und eine zu starke Zersetzung von PDA vermieden werden. Das Azeotrop fällt als Destillat an und wird nach Kondensation in eine wässrige und eine organische Phase getrennt, woraufhin die organische Phase als Rücklauf in die Destillationsvorrichtung zurückgeführt wird und die wässrige Phase aus dem System entfernt wird. Die hohe Dichte des ODB und damit die große Differenz zur Dichte von Wasser wird dabei als vorteilhaft für die Phasentrennung beschrieben.

Nachteilig an diesem Verfahren ist, dass ein Teil des PDA zusammen mit dem Azeotrop über Kopf destilliert wird. Aufgrund der guten Löslichkeit von PDA in Wasser verbleibt dieses nur zum Teil in der organischen Phase, während der andere Teil mit der wässrigen Phase die Destillationsvorrichtung verlässt und sich nur mit erheblichem Aufwand zurückgewinnen ließe. Darüber hinaus wirkt das PDA im Destillat als Lösungsvermittler, so dass auch ein erhöhter Anteil an ODB im Wasser verbleibt.

Dem Fachmann sind andere Verfahren zur Trocknung von organischen Substanzen bekannt, die jedoch ihrerseits bei der industriellen Anwendung zur Trocknung von PDA mit Nachteilen behaftet sind.

So kann eine Trocknung beispielsweise durch Membranverfahren erfolgen. Hierzu können beispielsweise Zeolithmembranen verwendet werden. Bevorzugte Verfahren sind dabei die Pervaporation oder die Dampfpermeation. Eine andere Möglichkeit stellt die Behandlung mit Trocknungsmitteln dar. Bevorzugt werden dabei feste Trocknungsmittel verwendet, die sich leicht durch Filtration wieder vom zu Trocknenden PDA separieren lassen. Bevorzugt sind solche Trocknungsmittel, die sich anschließend beispielsweise thermisch oder durch eine Vakuumbehandlung regenerieren lassen, wie beispielsweise Zeolithe, insbesondere Molekularsiebe. Besonders geeignet sind Molekularsiebe mit Porenweiten von 3 Å bis 4 Å bevorzugt 4 Å. Weitere Möglichkeiten bestehen darin, das PDA zu kristallisieren, gegebenenfalls mehrfach umzukristallisieren, ein Strippgas wie beispielsweise trockenen Stickstoff, bevorzugt zuvor erwärmten trockenen Stickstoff, einzuleiten oder eine Extraktion mit einem organischen Lösungsmittel durchzuführen. Auch Kombinationen dieser genannten Verfahren sind möglich.

Aufgabe der vorliegenden Erfindung war es, ein verbessertes Verfahren zur Trocknung von 1,5-Diaminopentan bereitzustellen, mit dem sich die Nachteile des Standes der Technik vermeiden lassen und das sich einfach industriell anwenden und skalieren lässt.

Diese Aufgabe konnte gelöst werden durch ein Verfahren zur Trocknung von 1,5-Diaminopentan umfassend die folgenden Schritte:
a) Bereitstellen mindestens eines Stoffgemisches enthaltend 1,5-Diaminopentan und Wasser,
b) Destillieren des Stoffgemisches in mindestens einer Destillationskolonne unter Erhalt einer getrockneten 1,5 -Diaminopentanzusammensetzung,
dadurch gekennzeichnet, dass die Destillation in Schritt b) azeotrop unter Verwendung eines Schleppmittels enthaltend Monochlorbenzol durchgeführt wird.

Das erfindungsgemäße Verfahren hat folgende Vorteile:
- Das Wasserazeotrop ist im Wesentlichen frei von 1,5-Diaminopentan
- Das Wasserazeotrop bildet nach Kondensation zwei Phasen, wobei die wässrige Phase nur sehr geringe Mengen an Monochlorbenzol (im Folgenden auch als "Monochlorbenzol" oder "MCB" bezeichnet) enthält
- Je nach Fahrweise kann das 1,5-Diaminopentan nicht nur wasserfrei, sondern auch frei von Monochlorbenzol erhalten werden.

Bevorzugt bedeuten erfindungsgemäß die Ausdrücke "umfassend" oder "enthaltend", "im Wesentlichen bestehend aus" und besonders bevorzugt "bestehend aus".

*Gehaltsangaben organischer Verbindungen* beziehen sich im Rahmen der vorliegenden Erfindung, sofern nicht anders angegeben, auf per Gaschromatographie bestimmte Werte und beziehen sich auf jeweils auf die Masse. Sofern nicht ausdrücklich anders angegeben, handelt es sich also bei Prozentangaben um Gewichtsprozente und bei ppm-Angaben um Gewichts-ppm. Die quantitative Auswertung von Gaschromatogrammen, gegebenenfalls mit Hilfe eines internen Standards, ist dem Fachmann bekannt. Gegebenenfalls erforderliche Methoden zur Bestimmung des *Wassergehalts* sind dem Fachmann ebenfalls bekannt. Die Methoden des Standes der Technik können auch im Rahmen der vorliegenden Erfindung angewandt werden. Im Zweifelsfall ist der per Karl-Fischer-Titration bestimmte Wassergehalt maßgeblich. Die Anwesenheit von Aminen kann zu schleppenden Endpunkten führen. In solchen Fällen ist der per Karl-Fischer-Titration nach Pufferung mit wasserfreier Benzoesäure bestimmte Wert maßgeblich. Das Verfahren unter Pufferung mit Benzoesäure ist dem Fachmann ebenfalls bekannt. Zur Karl-Fischer-Titration allgemein, siehe Jander, Jahr, *Maßanalyse,* 17. Aufl., de Gruyter, Berlin (2009), S. 279 bis S. 282).

Unter *"Destillation"* wird vorliegend ein thermisches Trennverfahren verstanden, das zur Gewinnung von verdampfbaren Stoffen, vorzugsweise Flüssigkeiten aus einem Stoffgemisch dient. Die abgetrennten Dämpfe werden anschließend in der Regel durch Kondensation niedergeschlagen. Der Begriff umfasst insbesondere die wiederholte Verdampfung und Kondensation unter Verwendung einer Kolonne (Destillationskolonne) mit einer Mehrzahl an Trennstufen. Solche Verfahren, bei denen mehrere Destillationsschritte in einer Kolonne hintereinandergeschaltet werden, werden strenggenommen als Rektifikation bezeichnet, sollen hier im Weiteren aber vereinfacht ebenfalls als Destillation bezeichnet werden. Der Vorteil solcher Verfahren ist der hohe Trenneffekt und die Möglichkeit die Anlage kontinuierlich zu betreiben.

Eine *"Destillationsvorrichtung"* ist ein Apparat, der geeignet ist, ein solches thermisches Trennverfahren durchzuführen, also beispielsweise Siebbodenkolonnen, Packungskolonnen, Füllkörperkolonnen, Glockenbodenkolonnen oder auch einstufige Verdampfer wie beispielsweise Fallfilmverdampfer, Dünnschichtverdampfer, Flashverdampfer, Mehrphasenwendelrohrverdampfer, Natur- oder Zwangsumlaufverdampfer. Die einzelnen Verfahrensschritte werden im Folgenden näher erläutert.

Zur Durchführung des erfindungsgemäßen Verfahrens wird zunächst ein Stoffgemisch enthaltend 1,5-Diaminopentan (im Folgenden auch als "PDA" oder "Pentamethylendiamin" bezeichnet) und Wasser bereitgestellt. Bevorzugt hat das bereitgestellte Stoffgemisch einen Wassergehalt von mehr als 500 ppm, besonders bevorzugt von 0,08 Gew.-% bis 10,0 Gew.-%, ganz besonders bevorzugt von 0,1 Gew.-% bis 5,0 Gew.-% und am meisten bevorzugt von 0,15 bis 3,0 Gew.-%, bezogen auf das in dem Stoffgemisch enthaltene Pentamethylendiamin. Bevorzugt enthält das bereitgestellte Stoffgemisch > 30 Gew.-%, besonders bevorzugt > 50 Gew.-%, ganz besonders bevorzugt >70 Gew.-% und am meisten bevorzugt >90 Gew.-% an Pentamethylendiamin bezogen auf das Gesamtgewicht des Stoffgemisches.

Das Pentamethylendiamin in dem bereitgestellten Stoffgemisch kann beispielsweise aus einem Recycling-Prozess von Polyurethanen, Polythiourethanen, Polyharnstoffen, Polyisocyanuraten oder Polyamiden basierend auf Pentamethylendiisocyanat bzw. Pentamethylendiamin stammen. Ein weiteres Beispiel für die Herkunft des Pentamethylendiamin ist dessen biotechnologische Herstellung, beispielsweise durch Fermentation geeigneter Vorläuferverbindungen. Nach der Herstellung des Pentamethylendiamins im eigentlichen Sinne durchläuft dieses üblicherweise eine destillative Reinigung, gegebenenfalls gekoppelt mit anderen Arten der Aufarbeitung, wie beispielsweise Extraktion oder Kristallisation. Da ein großer Teil des industriell hergestellten PDA zur Polyamid-Herstellung verwendet wird, für die weniger strenge Anforderungen an den Wassergehalt gelten als für die Isocyanat-Herstellung, wird dabei teilweise auf eine aufwändige Trocknung bis zu Wassergehalten <500 ppm verzichtet. Auch ist es möglich, dass ein zuvor bereits getrocknetes PDA durch Kontakt beispielsweise mit Luftfeuchtigkeit wieder Wasser aufnimmt, so dass der Wassergehalt letztlich die genannte Grenze von 500 ppm überschreitet und es nicht mehr zum Einsatz in einer Phosgenierungsreaktion geeignet ist. Das vorliegende Verfahren kann genutzt werden, den Wassergehalt eines solchen PDA auf akzeptable Werte für die Phosgenierung zu senken.

Die Destillation kann erfindungsgemäß diskontinuierlich als Batch-Destillation oder kontinuierlich durchgeführt werden, wobei jeweils ein Azeotrop enthaltend Wasser und Monochlorbenzol vorzugsweise als gasförmiger Brüdenstrom aus der Destillationsvorrichtung entnommen wird. Die Destillation erfolgt vorzugsweise bei einem Druck ≤ 250 kPa, bevorzugt ≤ 101,3 kPa, besonders bevorzugt ≤ 60 kPa, gemessen am Kopf der Destillationskolonne.

Es ist erfindungswesentlich, dass die Destillation in Gegenwart eines Schleppmittels enthaltend Monochlorbenzol erfolgt. Hierzu ist es beispielsweise möglich, das Monochlorbenzol dem 1,5-Pentandiamin vor dem Einbringen in die Destillationsvorrichtung beizumischen oder das Monochlorbenzol separat in die Destillationsvorrichtung einzubringen, so dass es erst dort zum Kontakt von 1,5-Pentandiamin und Monochlorbenzol kommt.

Bevorzugt kommt als Schleppmittel im Wesentlichen reines Monochlorbenzol zum Einsatz. Es ist aber auch möglich, dass es sich bei dem Schleppmittel enthaltend Monochlorbenzol um eine Schleppmittelmischung aus Monochlorbenzol und mindestens einem weiteren Lösungsmittel wie beispielsweise Toluol, Xylol oder o-Dichlorbenzol handelt. Bevorzugt sind in dem Fall Schleppmittelmischungen aus Monochlorbenzol und o-Dichlorbenzol. Bevorzugt enthält das Schleppmittel mindestens 5 Gew.-% Monochlorbenzol, besonderes bevorzugt mindestens 20 Gew.-% Monochlorbenzol und ganz besonders bevorzugt mindestens 40 Gew.-% Monochlorbenzol. Der Einsatz solcher Mischungen kann vorteilhaft sein, wenn eine spätere Phosgenierung auch in Gegenwart eines entsprechenden Lösungsmittelgemisches erfolgen soll. Besonders bevorzugt besteht das Schleppmittel aus mindestens technisch reinem Monochlorbenzol. In diesem Fall, lassen sich die entsprechenden Mischungen bei Bedarf nach der Trocknung problemlos einstellen.

Um eine thermische Zersetzung des PDA zu vermeiden oder zumindest zu minimieren, ist es bevorzugt, die Destillation bei einer Sumpftemperatur ≤ 200 °C, bevorzugt ≤ 160 °C, besonders bevorzugt ≤ 140°C und ganz besonders bevorzugt ≤ 130 °C durchzuführen. Die Sumpftemperatur lässt sich über den Druck im inneren der Kolonne einstellen. Bevorzugt liegt die Sumpftemperatur ≥ 80 °C, besonders bevorzugt ≥ 100 °C, ganz besonders bevorzugt ≥ 110 °C und am meisten bevorzugt ≥ 120 °C. Dies ermöglicht eine effiziente Kondensation der Destillationsbrüden.

Die Brüden aus der Destillation werden vorzugsweise nach Austritt aus der Kolonne in einem Kondensator zumindest teilweise kondensiert. Die Temperatur des Kondensats liegt bevorzugt im Bereich von 5 °C bis 115 °C, besonders bevorzugt im Bereich von 10 °C bis 100 °C und ganz besonders bevorzugt im Bereich von 20 °C bis 70 °C. Die dabei kondensierten Bestandteile der Brüden werden anschließend einer Phasentrennung in mindestens einem Phasentrennapparat unterzogen. Um die Phasentrennung in eine überwiegend wässrige und eine überwiegend organische Phase zu verbessern, ist es bei höheren Kondensationstemperaturen bevorzugt, das Kondensat vor Eintritt in den Phasentrennapparat auf eine Temperatur im Bereich von 5 °C bis 65 °C, bevorzugt im Bereich von 10 °C bis 50 °C und besonders bevorzugt im Bereich von 15 °C bis 45 °C zu kühlen. Als Phasentrennapparate können alle dem Fachmann geläufigen Ausführungsformen eingesetzt werden. Bevorzugt wird die Trennung in einem Schwerkraftabscheider, beispielsweise einem Settler durchgeführt. Bei Bedarf können als Koaleszenzhilfe entsprechende Einbauten wie Platten, Coalescer Pads oder Elektrophoresezellen verwendet werden oder Zuschlagstoffe, wie beispielsweise Salze zu der kondensierten Flüssigkeit gegeben werden.

Die in der Phasentrennung erhaltene organische Phase wird zumindest teilweise, vorzugsweise vollständig als Rücklauf in die Destillationsapparatur zurückgeführt, während die erhaltene wässrige Phase zumindest teilweise, vorzugsweise vollständig aus dem System entfernt und gegebenenfalls weiter aufbereitet wird, um wertvolle Inhaltsstoffe zurückzugewinnen oder eine Entsorgung zu vereinfachen.

Die erfindungsgemäße Trocknung kann beispielsweise unter Nutzung einer herkömmlichen Destillationsvorrichtung erfolgen. Diese umfasst bevorzugt mindestens eine, besonders bevorzugt genau eine Kolonne, mit 2 bis 50, bevorzugt 5 bis 30 und besonders bevorzugt 10 bis 25 theoretischen Trennstufen. Vorzugsweise befinden sich in der mindestens einen Kolonne sogenannte trennwirksame Einbauten zur Vergrößerung der Stoffaustauschfläche. Diese Einbauten können beispielsweise verschiedene Arten von Stoffaustauschböden (Glockenböden, Siebböden, Ventilböden, usw.), Packungen mit ungeordneter Schüttung von Füllkörpern oder sogenannte Strukturpackungen sein, wobei letztere üblicherweise einen niedrigen Druckverlust aufweisen und deshalb bevorzugt sind. Die Destillation erfolgt vorzugsweise bei einem Druck ≤ 250 kPa, bevorzugt ≤ 101,3 kPa, besonders bevorzugt ≤ 60 kPa, gemessen am Kopf der Destillationskolonne. Ein geringer Druck ermöglicht eine Destillation bei niedrigeren Temperaturen und reduziert die Gefahr von Ausbeuteverlusten aufgrund von Nebenreaktionen des 1,5-Pentandiamins. Er geht jedoch auch stets mit einer geringen Gasdichte in der Destillationsapparatur einher, so dass für hohe Durchsätze bei geringem Druck sehr große Apparate erforderlich werden. Außerdem erfordert die Kondensation der Brüden bei geringem Druck tiefere Temperaturen, so dass der Aufwand für das Abkühlen der Brüden steigt. Es ist deshalb bevorzugt, die Kolonne mit einem Druck ≥5 kPa, besonders bevorzugt ≥10 kPa und ganz besonders bevorzugt ≥20 kPa, gemessen am Kopf der Kolonne zu betreiben.

Erfolgt die Trocknung als diskontinuierliche Destillation (Batchdestillation), so ist es bevorzugt, wenn in dem zu destillierenden Gemisch zu Beginn der Destillation das Massenverhältnis von Monochlorbenzol zu PDA im Bereich von 1:100 bis 100:1, bevorzugt im Bereich von 1:10 bis 10: 1 und ganz besonders bevorzugt im Bereich von 1:2 bis 2:1 liegt. Vorzugsweise wird die Destillation dann so lange betrieben, bis der gewünschte Wassergehalt in der Destillationsblase erreicht wird, in der dann eine Mischung aus 1,5-Pentandiamin und Monochlorbenzol mit einem Wassergehalt von weniger als 500 ppm, bevorzugt weniger als 200 ppm und ganz besonders bevorzugt weniger als 100 ppm bezogen auf das enthaltene Pentamethylendiamin vorliegt. Sofern für den nachfolgenden Prozess ein lösungsmittelfreies Pentamethylendiamin benötigt wird, kann die Destillation bei verringertem Druck und/oder erhöhter Temperatur weiter fortgeführt werden, bis auch das Monochlorbenzol über Kopf abgetrennt wurde.

In einer bevorzugten Ausführungsform des Verfahrens erfolgt die Trocknung als kontinuierliche Destillation. In dieser Ausführungsform ist es möglich, das zu destillierende Stoffgemisch direkt in die Kolonne einzubringen und die Destillation erfolgt nach Erreichen eines stationären Zustands unter annähernd konstanten Bedingungen.

In einer besonders vorteilhaften Ausführungsform dieser kontinuierlichen Destillation umfasst die Kolonne dem Verstärkerteil auch einen Abtriebsteil, und es kann am Sumpf der Kolonne direkt in einem Schritt und damit sehr effizient ein weitgehend schleppmittelfreies getrocknetes 1,5-Pentandiamin entnommen werden. Dies ist insbesondere dann von Vorteil, wenn das getrocknete 1,5-Pentandiamin anschließend in einer Gasphasenphosgenierung zum 1,5-Pentandiisocyanat umgesetzt werden soll, wobei häufig zunächst auf Lösungsmittel im Amin verzichtet wird, beispielsweise um eine weitere Chlorierung des Lösungsmittels während der Phosgenierung zu vermeiden. Das Schleppmittel zirkuliert in dieser vorteilhaften Ausführungsform am Kopf der Destillationskolonne, das heißt, es verlässt die Kolonne als Azeotrop mit Wasser am Kopf und wird dann nach Kondensation und Phasentrennung als Rücklauf wieder in die Kolonne zurückgeführt. Die Verluste an Schleppmittel sind aufgrund der geringen Löslichkeit in der wässrigen Phase minimal, so dass im Grunde eine einmalige anfängliche Befüllung des Systems mit dem Schleppmittel ausreicht. Geringe unvermeidliche Verluste können bevorzugt ausgeglichen werden, indem man frisches Monochlorbenzol dem Rücklaufstrom beimischt oder dieses einfach in den Phasentrennapparat einbringt.

Zur Durchführung der kontinuierlichen azeotropen Destillation ist es bevorzugt, wenn das Verhältnis aus dem Gesamtmassenstrom an Monochlorbenzol in die Kolonne zu dem Massenstrom an PDA, der als Feed in die Kolonne gefahren wird, im Bereich von 100:1 bis 1:100, besonders bevorzugt im Bereich von 10:1 bis 1:10 und ganz besonders bevorzugt im Bereich von 1:1 bis 1:5 liegt. Der Gesamtmassenstrom an Monochlorbenzol in die Kolonne umfasst dabei sowohl Monochlorbenzol, welches zusammen mit dem PDA als Feed in die Kolonne gefahren wird als auch Monochlorbenzol, welches als Rücklauf auf die Kolonne gefahren wird, also die organische Phase aus der Phasentrennung nach Kondensation der Brüden, sowie gegebenenfalls separat in die Kolonne eingespeiste Monochlorbenzol enthaltenden Ströme.

Es ist möglich, die azeotrope Destillation in Kombination mit mindestens einem weiteren Trocknungsverfahren einzusetzen, wobei das mindestens eine weitere Trocknungsverfahren bevorzugt ein Membrantrennverfahren, eine Behandlung mit festen Trocknungsmitteln, eine Extraktion, eine Kristallisation und/oder das Durchleiten von Strippgas ist. Vorzugsweise erfolgt die Trocknung bei dieser Ausführungsform zunächst durch azeotrope Destillation und die in Schritt b) erhaltene getrocknete 1,5-Diaminopentanzusammensetzung wird dann mit Hilfe des weiteren Trocknungsverfahrens weiter getrocknet. Auf diese Weise sind 1,5-Diaminopentanzusammensetzungen mit einem besonders niedrigen Wassergehalt zugänglich. Das erfindungsgemäße Verfahren ist gleichbedeutend mit einem Verfahren zur Herstellung einer solchen getrockneten 1,5 -Diaminopentanzusammensetzung.

Ein weiterer Gegenstand der Erfindung ist ein Verfahren zur Herstellung von Pentamethylendiisocyanat durch Umsetzung von 1,5-Pentandiamin mit Phosgen, dadurch gekennzeichnet, dass das 1,5-Pentandiamin zuvor in einer azeotropen Destillation unter Verwendung eines Schleppmittels enthaltend Monochlorbenzol getrocknet wurde.

Die Phosgenierung des erfindungsgemäß getrockneten 1,5-Pentandiamins kann beispielsweise in der Gasphase erfolgen. Verfahren zur Gasphasenphosgenierung von aliphatischen Diaminen allgemein, aber auch von 1,5-Pentandiamin sind dem Fachmann aus dem Stand der Technik hinlänglich bekannt. Die Phosgenierung erfolgt bei Temperaturen im Bereich von 200 bis 600 °C mit einem Überschuss an Phosgen, gegebenenfalls in Gegenwart eines Inertgases bzw. Dämpfen eines inerten Lösungsmittels. Bevorzugt erfolgt die Phosgenierung wie in WO2016042125 A1 beschrieben.

Nach der erfolgten Umsetzung in einem vorzugsweise zylindrischen Reaktionsraum wird Reaktionsgemisch bevorzugt durch selektive Kondensation in einem inerten Lösungsmittel wie beispielswiese Monochlorbenzol oder o-Dichlorbenzol von dem gebildeten Pentamethylendiisocyanat befreit, welches anschließend in einer mehrstufigen Destillation zum reinen Pentamethylendiisocyanat aufbereitet wird.

In einer weiteren Ausführungsform erfolgt die Phosgenierung des erfindungsgemäß getrockneten 1,5-Diaminopentans in Schritt in der flüssigen Phase. Die Reaktion kann dann auf verschiedene Weise ausgeführt werden. Entweder wird das Diamin direkt mit einem Überschuss an Phosgen in einem inerten flüssigen Medium, vorzugsweise in einer zweistufigen, sogenannten Kalt-Heiß-Phosgenierung, umgesetzt (Basenphosgenierung) oder es wird zunächst durch Umsetzung mit Chlorwasserstoffgas oder Kohlendioxid in einem inerten flüssigen Medium das entsprechende Salz überführt und dann mit überschüssigem Phosgen ähnlich dem Heißphosgenierungsschritt der Basenphosgenierung umgesetzt (Hydrochlorid- bzw. Carbaminatphosgenierung). Als flüssiges Medium eignet sich insbesondere das während der Trocknung eingesetzte Schleppmittel.

Sowohl bei der Basenphosgenierung als auch bei der Amin-Hydrochlorid- bzw. der CarbaminatPhosgenierung wird nach Beendigung der Reaktion das verbliebene Phosgen und Chlorwasserstoffgas bevorzugt mit einem Inertgas, vorzugsweise mit Stickstoff, ausgeblasen. Bei Bedarf kann eine Filtration erfolgen, um gegebenenfalls vorhandene Feststoffe wie unreagierte Amin-Hydrochloride zu entfernen. Das gebildete Pentamethylendiisocyanat wird vorzugsweise anschließend in einer mehrstufigen Destillation zum reinen Pentamethylendiisocyanat aufbereitet.

Auf dieses Weise hergestelltes Pentamethylendiisocyanat ist zur Herstellung von LackPolyisocyanaten, also oligomeren Polyisocyanaten enthaltend Allophanatgruppen, Biuretgruppen, Isocyanuratgruppen, Uretdiongruppen, Urethangruppen und/oder Iminooxadiazindiongruppen bestens geeignet. Somit ist die Verwendung eines erfindungsgemäß hergestellten 1,5-Diisocyanatopentans zur Herstellung von oligomeren Polyisocyanaten, enthaltend Allophanatgruppen, Biuretgruppen, Isocyanuratgruppen, Uretdiongruppen, Urethangruppen und/oder Iminooxadiazindiongruppen, ein weiterer Gegenstand der Erfindung.

### Beispiele

### Beispiel 1 (Vergleichsbeispiel):

In Anlehnung an die Offenbarung aus CN216571619 U wurde eine Destillationsvorrichtung bestehend aus Destillationsblase, Kolonne, Kondensator und Trennflasche als Modell berechnet. Für die Kolonne wurde ein Verstärkerteil mit 20 Trennstufen angenommen und die Temperatur in der Destillationsblase wurde auf maximal 130 °C festgelegt. Ein Überschreiten dieser Temperatur wurde durch eine Absenkung des Drucks verhindert. Eine Mischung bestehend aus 50,0 Gew.-% PDA, 1,0 Gew.-% Wasser und 49,0 Gew.-% ODB wurde als Batchdestillation so lange destilliert, bis in der Destillationsblase der Wassergehalt auf 50 Massen-ppm gesenkt wurde. Die am Kopf der Kolonne entnommenen Brüden wurden kondensiert und einer Phasentrennung unterzogen, wobei die überwiegend organische Phase als Rücklauf in die Kolonne zurückgeführt wurde und die überwiegend wässrige Phase ausgeschleust wurde. Am Ende der Destillation stellte sich am Kopf der Kolonne ein Druck von ca. 225 mbar(a) ein. Die im Verlauf der Destillation ausgeschleuste wässrige Phase enthielt neben ca. 84,8 Gew.-% Wasser auch noch ca. 8,0 Gew.-% ODB und 7,2 Gew.-% PDA. Dies entspricht einem Verlust von ca. 0,17 Gew.-% des eingesetzten PDA und ca. 0,2 Gew.-% der eingesetzten Menge des Schleppmittels ODB.

### Beispiel 2 (erfindungsgemäß):

Die Modellberechnung aus Beispiel 1 wurde wiederholt, wobei diesmal eine Mischung bestehend aus 50,0 Gew.-% PDA, 1,0 Gew.-% Wasser und 49,0 Gew.-% MCB vorgelegt wurde. Im Gegensatz zu der Berechnung in Beispiel 1 stellte sich in diesem Fall am Ende der Destillation ein Kopfdruck von 565 mbar(a) ein. Die im Verlauf der Destillation ausgeschleuste wässrige Phase enthielt neben 99,8 Gew.-% Wasser noch ca. 0,2 Gew.-% MCB und weniger als 1 Gew.-ppm an PDA. Es erfolgte also praktisch kein Verlust von PDA mit dem ausgeschleusten Wasser und nur ein sehr geringer Verlust von weniger als 0,01 Gew.-% der eingesetzten Menge des Schleppmittels MCB.

### Beispiel 3 (erfindungsgemäß)

Die Modellberechnung aus Beispiel 1 wurde wiederholt, wobei diesmal eine Mischung bestehend aus 94,3 Gew.-% PDA, 1,9 Gew.-% Wasser und 3,4 Gew.-% MCB vorgelegt wurde. Die Destillation verlief in dieser Simulation ähnlich wie in Beispiel 2, mit dem Unterschied, dass der Kopfdruck zum Ende der Destillation bis auf 238 mbar abgesenkt werden musste, um die Sumpftemperatur von 130 °C nicht zu überschreiten. Die im Verlauf der Destillation ausgeschleuste wässrige Phase enthielt neben 99,9 Gew.-% Wasser noch ca. 0,1 Gew.-% MCB und weniger als 1 Gew.-ppm an PDA. Es erfolgte also praktisch kein Verlust von PDA mit dem ausgeschleusten Wasser und nur ein sehr geringer Verlust von weniger als 0,1 Gew.-% der eingesetzten Menge des Schleppmittels MCB. Aufgrund der wesentlich geringeren Einsatzmenge an Schleppmittel war der absolute Verlust an Schleppmittel ähnlich wie in Beispiel 2.

### Beispiel 4 (erfindungsgemäß)

Noch einmal wurde die Modellberechnung aus Beispiel 1 wiederholt, wobei diesmal eine Mischung bestehend aus 50,0 Gew.-% PDA, 1,0 Gew.-% Wasser und jeweils 24,5 Gew.-% MCB und ODB vorgelegt wurde. Es stellte sich am Ende der Destillation ein Kopfdruck von 405 mbar(a) ein und die im Verlauf der Destillation ausgeschleuste wässrige Phase enthielt neben 99,8 Gew.-% Wasser noch ca. 0,2 Gew.-% MCB. Sie enthielt weniger als 1 Gew.-ppm an PDA und ODB. Es erfolgte also praktisch kein Verlust von PDA oder ODB und nur ein sehr geringer Verlust von weniger als 0,01 Gew.-% der eingesetzten Menge des Schleppmittels MCB.

### Beispiel 5 (Vergleichsbeispiel)

Die Modellberechnung aus Beispiel 1 wurde wiederholt, wobei diesmal für die Kolonne ein Verstärkerteil von 40 Trennstufen angenommen wurde. Am Ende der Destillation stellte sich am Kopf der Kolonne wieder ein Druck von ca. 225 mbar(a) ein. Die im Verlauf der Destillation ausgeschleuste wässrige Phase enthielt neben ca. 93,1 Gew.-% Wasser auch noch ca. 6,5 Gew.-% ODB und 0,4 Gew.-% PDA. Dies entspricht einem Verlust von ca. 0,01 Gew.-% des eingesetzten PDA und gut 0,1 Gew.-% des eingesetzten Schleppmittels ODB.

### Beispiel 6 (erfindungsgemäß)

In diesem Beispiel wurde die Trocknung als kontinuierliche azeotrope Destillation gerechnet. Als Eingangsstrom wurde ein Strom bestehend aus 50 Gew.-% MCB, 49 Gew.-% PDA und 1 Gew.-% Wasser vorgegeben. Als Destillationsvorrichung wurde eine Kolonne mit Verdampfer und 31 Trennstufen verwendet, der Feed erfolgte auf der 15. Stufe. Die am Kopf der Kolonne entnommenen Brüden wurden bei 50 °C Kondensiert und in eine organische sowie eine wässrige Phase getrennt. Letztere wurde aus dem System ausgeschleust, während die organische Phase als Rücklauf auf den Kopf der Kolonne gegeben wurde. Die Heizleistung des Kolonnenverdampfers wurde so angepasst, dass der am Sumpf entnommene Produktstrom einen Wassergehalt von 100 Gew.-ppm bezogen auf das enthaltene PDA enthielt. Außerdem wurde der Kopfdruck der Kolonne auf 574 mbar(a) reduziert, so dass sich am Sumpf eine Temperatur von 130 °C einstellte. Der ausgeschleuste Wasserstrom enthielt weniger als 0,1 Gew.-% an MCB und weniger als 1 Gew.-ppm an PDA.

### Beispiel 7 (Vergleichsbeispiel)

In diesem Beispiel wurde die Trocknung als kontinuierliche azeotrope Destillation gerechnet. Als Eingangsstrom wurde ein Strom bestehend aus 50 Gew.-% ODB, 49 Gew.-% PDA und 1 Gew.-% Wasser vorgegeben. Als Destillationsvorrichung wurde eine Kolonne mit Verdampfer und 31 Trennstufen verwendet, der Feed erfolgte auf der 15. Stufe. Die am Kopf der Kolonne entnommenen Brüden wurden bei 50 °C Kondensiert und in eine organische sowie eine wässrige Phase getrennt. Letztere wurde aus dem System ausgeschleust, während die organische Phase als Rücklauf auf den Kopf der Kolonne gegeben wurde. Die Heizleistung des Kolonnenverdampfers wurde so angepasst, dass der am Sumpf entnommene Produktstrom einen Wassergehalt von 100 Gew.-ppm bezogen auf das enthaltene PDA enthielt. Der Kopfdruck der Kolonne musste auf 227 mbar(a) reduziert werden, um die gleiche niedrige Sumpftemperatur von 130 °C zu erreichen wie in Beispiel 6. Der ausgeschleuste Wasserstrom enthielt neben 94,3 Gew.-% Wasser auch noch ca. 0,9 Gew.-% PDA, entsprechend ca. 0,02% der im Feed eingesetzten Menge sowie 4,8 Gew.-% ODB, entsprechend .ca. 0,1 % des im Feed eingesetzten Schleppmittels ODB.

### Beispiel 8 (erfindungsgemäß)

Die Modellberechnung aus Beispiel 6 wurde derart verändert, dass am Sumpf der Destillationsvorrichtung ein PDA entnommen wurde, welches praktisch frei von Wasser und dem Schleppmittel MCB war. Dazu wurde einerseits das MCB aus dem Zulaufstrom der Kolonne entfernt, so dass hier ein Gemisch bestehend aus 98 Gew.-% PDA und 2 Gew.-% Wasser auf der 15. Stufe in die Kolonne gefahren wurde. Für die Phasentrennung wurde von einer anfänglichen Füllung mit MCB ausgegangen, so dass das MCB über den Rücklauf in die Kolonne gelangte, dort wieder verdampfte und sich auf diese Weise letztlich ein MCB-Kreislauf am Kopf der Kolonne über die Phasentrennung ausbildete. Verluste an MCB wurden durch eine kleine Zugabe von MCB in den Rücklaufstrom zur Kolonne ausgeglichen. Das Sumpfprodukt PDA enthielt in Summe ca. 1 Gew.-ppm an Wasser und MCB. Um die Sumpftemperatur von 130 °C einzuhalten, wurde der Kopfdruck der Kolonne auf 214 mbar(a) gesenkt. Der ausgeschleuste Wasserstrom enthielt weniger als 0,1 Gew.-% an MCB und weniger als 1 Gew.-ppm an PDA.

## Patentansprüche

1. Verfahren zur Trocknung von 1,5-Diaminopentan umfassend die folgenden Schritte:
a) Bereitstellen mindestens eines Stoffgemisches enthaltend 1,5-Diaminopentan und Wasser,
b) Destillieren des Stoffgemisches in mindestens einer Destillationskolonne unter Erhalt von Brüden sowie einer getrockneten 1,5-Diaminopentanzusammensetzung,
**dadurch gekennzeichnet, dass** die Destillation in Schritt b) azeotrop unter Verwendung eines Schleppmittels enthaltend Monochlorbenzol durchgeführt wird.

2. Verfahren gemäß Anspruch 1, **dadurch gekennzeichnet, dass** die azeotrope Destillation diskontinuierlich oder kontinuierlich durchgeführt wird.

3. Verfahren gemäß einem der Ansprüche 1 oder 2, **dadurch gekennzeichnet, dass** die azeotrope Destillation bei einem Druck ≤ 250 kPa, bevorzugt ≤ 101,3 kPa, besonders bevorzugt ≤ 60 kPa, gemessen am Kopf der Destillationskolonne, durchgeführt wird.

4. Verfahren gemäß einem der Ansprüche 1 bis 3, **dadurch gekennzeichnet, dass** das eingesetzte Schleppmittel mindestens 5 Gew.-%, bevorzugt mindestens 20 Gew.-% und besonders bevorzugt mindestens 40 Gew.-% Monochlorbenzol enthält oder dass das Schleppmittel aus mindestens technisch reinem Monochlorbenzol besteht.

5. Verfahren gemäß einem der Ansprüche 1 bis 4, **dadurch gekennzeichnet, dass** die Sumpftemperatur ≥ 80 °C, bevorzugt ≥ 100 °C, besonders bevorzugt ≥ 110 °C und ganz besonders bevorzugt ≥ 120 °C beträgt.

6. Verfahren gemäß einem der Ansprüche 1 bis 5, **dadurch gekennzeichnet, dass** die Brüden aus der Destillation zumindest teilweise kondensiert werden und das erhaltene Kondensat bei einer Temperatur im Bereich von 5 °C bis 65 °C, bevorzugt im Bereich von 10 °C bis 50 °C und besonders bevorzugt im Beriech von 15 °C bis 45 °C in eine überwiegend wässrige und eine überwiegend organische Phase getrennt werden.

7. Verfahren gemäß einem der Ansprüche 1 bis 6, **dadurch gekennzeichnet, dass** die azeotrope Destillation in Kombination mit mindestens einem weiteren Trocknungsverfahren eingesetzt wird, wobei das mindestens eine weitere Trocknungsverfahren ein Membrantrennverfahren, eine Behandlung mit festen Trocknungsmitteln, eine Extraktion, eine Kristallisation und/oder das Durchleiten von Strippgas ist.

8. Verfahren gemäß einem der Ansprüche 1 bis 7, **dadurch gekennzeichnet, dass** in Schritt a) bereitgestellte Stoffgemisch einen Wassergehalt von mehr als 500 ppm, bevorzugt von 0,08 Gew.-% bis 10 Gew.-%, besonders bevorzugt von 0,1 Gew.-% bis 5 Gew.-% und ganz besonders bevorzugt von 0,15 Gew.-% bis 3 Gew.-%, bezogen auf das im Stoffgemisch enthaltene Pentamethylendiamin hat.

9. Verfahren gemäß einem der Ansprüche 1 bis 8, **dadurch gekennzeichnet, dass** das 1,5-Diaminopentan in dem in Schritt a) bereitgestellten Stoffgemisch aus einem Recycling-Prozess stammt und/oder biotechnologisch hergestellt wurde.

10. Verfahren gemäß einem der Ansprüche 1 bis 9, **dadurch gekennzeichnet, dass** in Schritt a) bereitgestellte Stoffgemisch zumindest eine Teilmenge des als Schleppmittel in Schritt b) verwendeten Monochlorbenzols enthält.

11. Verfahren zur Herstellung von 1,5-Diisocyanatopentan, umfassend Flüssigphasenphosgenierung oder Gasphasenphosgenierung der gemäß einem der Ansprüche 1 bis 10 hergestellten getrockneten 1,5-Diaminopentanzusammensetzung.

12. Verwendung eines gemäß Anspruch 11 hergestellten 1,5-Diisocyanatopentans zur Herstellung von oligomeren Polyisocyanaten, enthaltend Allophanatgruppen, Biuretgruppen, Isocyanuratgruppen, Uretdiongruppen, Urethangruppen und/oder Iminooxadiazindiongruppen.
